# EUROPEAN PATENT APPLICATION

(11) **EP 2 248 521 A1**
(43) Date of publication of application: **10.11.2010**
(21) Application number: 10172176.9
(22) Date of filing: 27.10.2005
(51) Int. Cl.: A61K 31/416, A61P 27/00, A61K 31/00

(54) **Inhibitors of jun n-terminal kinases for treating glaucomatous retinopathy and ocular diseases**

(30) Priority: 29.10.2004 US 623755 P
(62) Divisional of application: 05824291.8
(71) Applicant: Alcon, Inc., 6331 Hünenberg (CH)
(72) Inventor: Pang, Iok-hou, Grand Prairie, TX 75052 (US); Clark, Abbot F., Arlington, TX 76017 (US)
(74) Representative: Gates, Marie Christina Esther

(57) **Abstract**

Compositions and methods for the treatment of glaucoma and other ocular diseases are disclosed. The compositions and methods are particularly directed to the use inhibitors of Jun N-terminal kinases (JNK), such as SP600125 in the treatment of glaucoma and other ocular diseases.

## Description

### BACKGROUND OF THE INVENTION

### 1. Field of the Invention

The present invention relates generally to the field of ocular neuroprotection and more specifically to the use of inhibitors of Jun N-terminal kinases (JNK) to treat glaucomatous retinopathy and other ocular diseases.

### 2. Description of the Related Art

Many pathological changes in the eye, such as glaucoma, acute ischemic optic neuropathy, macular degeneration, retinitis pigmentosa, retinal detachment, retinal tears or holes, and other ischemic retinopathies or optic neuropathies, cause injury or death of retinal neurons, which can lead to loss of vision. For example, primary open-angle glaucoma (POAG) is a progressive disease leading to optic nerve damage and ultimately blindness. The cause of this disease has been the subject of extensive studies for many years, but is still not fully understood. Glaucoma results in the neuronal degeneration of the retina and optic nerve. Even under optimal medical care and surgical treatment, it is still associated with a gradual loss of retinal ganglion cells (RGC), which causes a decline of visual function (Van Buskirk *et al.* (1993); Schumer *et al.* (1994)).

An abnormal increase in intraocular pressure (IOP) is a major risk factor of glaucoma. Currently, the only available treatment for glaucoma is to lower IOP either by medication or surgery. Lowering IOP is effective in slowing the development of POAG and delaying its damaging effects. Nonetheless, the loss of visual field in glaucoma patients does not always correlate with IOP, and lowering IOP alone does not completely stop the disease process. This implies that pressure may not be the only cause of glaucomatous retinopathy and optic neuropathy. Additional mechanisms, especially those existing in the optic nerve head and retina, likely contribute to the death of RGC.

Several mechanisms of glaucomatous retinopathy have been hypothesized. None alone seems sufficient to explain the wide spectrum and patterns of pathological changes usually observed in glaucoma patients. It is probable that glaucoma involves more than one etiology and different mechanisms are manifested in different patients and/or different stages of the disease. Some of the more important proposals are: deprivation of neurotrophic factors, vascular abnormality (ischemia), and glutamate toxicity. These mechanisms eventually lead to apoptosis of the RGC (Clark & Pang (2002)).

The same mechanisms have been proposed to be involved in other ocular diseases. For example, a decrease in neurotrophic factors is associated with a rat model of retinitis pigmentosa (Amendola *et al.* (2003)). Introduction of certain neurotrophic factors to the retina can reduce retinal damages related to retinitis pigmentosa (Tao *et al.* (2002)), retinal detachment (Hisatomi *et al.,* (2002); Lewis *et al.* (1999)), and experimental macula degeneration (Yamada *et al.* (2001)). Retinal ischemia is involved in acute ischemic optic neuropathy, macular degeneration (Harris *et al.* (1999)), and other ischemic retinopathies or optic neuropathies. Similarly, glutamate toxicity may contribute to the retinal damages seen in retinal detachment (Sherry & Townes-Anderson (2000)).

Currently, no available therapy for glaucoma seeks to interrupt the mechanisms by which the ocular tissues are damaged in the disease process. What is needed is a glaucoma treatment that addresses the underlying pathological cause of the disease and thereby provides protection from retinal ganglion cell loss or damage

### SUMMARY OF THE INVENTION

The present invention overcomes these and other drawbacks of the prior art by providing compositions and methods for treating glaucoma and other ocular diseases aimed at affecting the mechanisms causing damage to the ocular tissues. The compositions and methods comprise at least one inhibitor of JNK for the treatment of compromised retinal tissue related to ocular diseases, such as glaucoma, acute ischemic optic neuropathy, macular degeneration, retinitis pigmentosa, retinal detachment, retinal tears or holes, and other ischemic retinopathies or optic neuropathies.

### BRIEF DESCRIPTION OF THE DRAWINGS

The following drawings form part of the present specification and are included to further demonstrate certain aspects of the present invention. The invention may be better understood by reference to these drawings in combination with the detailed description of specific embodiments presented herein.
**FIG. 1****.** Effect of SP600125 on rat RGC survival with or without trophic factors, with or without glutamate (100 µM). The cells were cultured with the respective conditions for 3 days. Survival was quantified by counting all Thy-1 positive healthy cells.
**FIG. 2****.** Effect of SP600125 on ischemia/reperfusion-induced optic neuropathy. An optic nerve damage score of 1 represented no damage, and a score of 5 represented total damage. *: p < 0.05 versus the vehicle-treated group by Student's t-test.
**FIG. 3**. Effects of SP600125 on the survival of cultured adult rat RGC. The cells were treated with glutamate (100 µM) with or without SP600125 for 3 days.
**FIG. 4****.** Effects of SP600125 on the survival of cultured adult rat RGC. Selected trophic factors (bFGF, BDNF, CNTF) were withdrawn from all wells except the controls. The cells were treated with the indicated concentrations of SP600125 for 3 days. (TF = trophic factors).

### DETAILED DESCRIPTION OF PREFERRED EMBODIMENTS

The present invention is directed to compositions and methods for treating glaucoma and other ocular diseases, including acute ischemic optic neuropathy, macular degeneration, retinitis pigmentosa, retinal detachment, retinal tears or holes, and other ischemic retinopathies or optic neuropathies. The compositions comprise one or more inhibitor(s) of JNK in a pharmaceutically acceptable vehicle.

Jun N-terminal kinases (JNK) are a family of stress-activated protein kinases comprising of at least 10 isoforms created by alternative splicing of mRNA transcripts derived from three genes: JNK1, JNK2, and JNK3 (Gupta *et al.* (1996)). Activation of JNK is required for certain forms of stress-induced apoptosis (Toumier *et al.* (2000)), which leads to phosphorylation of a number of transcription factors and cellular proteins, particularly those associated with apoptosis (e.g., Bcl2, Bcl-X_{L}, p53, etc.). In cell culture, activation of JNK correlates with neuronal apoptosis induced by a variety of insults (Xia *et al.* (1995); Le-Niculescu *et al.* (1999)). JNK3 is required for sympathetic neuron death following trophic factor withdrawal (Bruckner *et al.* (2001)). Mice deficient in JNK3 are resistant to the hippocampal neurotoxicity induced by kainic acid (Yang *et al.* (1997)). Because of these neuroprotective actions, inhibitors of JNK have been proposed as treatment for degenerative diseases of the brain, such as, Alzheimer's disease, Parkinson's disease, stroke, and ischemia-induced brain dysfunction. In addition, because the JNK signaling pathway also regulates the activity and metabolism of some of the molecules involved in inflammation (Manning & Mercurio (1997)), JNK inhibitors were proposed as treatment for immune diseases, such as rheumatoid arthritis, asthma, chronic transplant rejection, inflammatory bowel disease, and multiple sclerosis. Other studies further indicate that JNK inhibitors may be useful as potential therapeutic agents for obesity, type 2 diabetes (Hirosumi *et al.* (2002)), and cancer (Adjei (2001)).

It is not obvious that JNK inhibitors, even with multiple pharmacological actions listed above, are useful in treating glaucoma. The reasons are as follows. (1) None of the above mentioned diseases have been shown to be associated with glaucoma or the aforementioned ocular diseases. (2) The usefulness of a drug in the brain does not predict its usefulness in the eye, since therapeutic agents useful for degenerative diseases in the brain do not always protect against glaucomatous apoptotic death of RGC or other ocular diseases. (3) Inflammation, immune abnormality, diabetes, obesity, or cancer is not widely accepted as an etiology of glaucoma or the aforementioned ocular diseases.

Unexpectedly, the present inventors discovered that a non-peptide JNK inhibitor, SP-600125, was protective against glutamate-induced or trophic factor withdrawal-induced death of a rat retinal neuron, the RGC, in culture. The present inventors also found that the compound was protective against ischemia/reperfusion-induced optic neuropathy in the rat. Since deprivation of trophic factors, ischemia, and glutamate toxicity were proposed as potential mechanisms of glaucoma and various ocular diseases, these data indicate that non-peptide JNK inhibitors are useful as therapeutic agents for the treatment or prevention of glaucoma and other ocular diseases, such as acute ischemic optic neuropathy, macular degeneration, retinitis pigmentosa, retinal detachment, retinal tears or holes, and other ischemic retinopathies or optic neuropathies.

As used herein, "inhibitors of JNK" refers to those compounds which can decrease the activity of JNK to 50% or lower of the control value. The potential inhibitory effect of compounds on JNK activity can be easily evaluated by those skilled in the art. Many JNK activity assay kits are commercially available, e.g., Stratagene catalog # 205140, Upstate catalog # 17-166, etc.

Examples of JNK inhibitors expected to be useful in the methods and compositions of the present invention include, but not are limited to, SP600125 and pharmacologically active compounds disclosed in patent applications numbers WO200035906, WO200035909, WO200035921, WO200064872, W0200112609, WO20011262 W0200123378, WO200123379, WO200123382, WO200147920, W0200191749, WO2002046170, WO2002062792, WO2002081475, W02002083648, W02003 024967; all of which are hereby incorporated by reference.

The methods comprise administering one or more JNK inhibitors to a human patient for the treatment of glaucoma and/or other ocular diseases, such as acute ischemic optic neuropathy, macular degeneration, retinitis pigmentosa, retinal detachment, retinal tears or holes, and other ischemic retinopathies or optic neuropathies.

The JNK inhibitors of the present invention may be contained in various types of pharmaceutical compositions, in accordance with formulation techniques known to those skilled in the art. In general, the JNK inhibitors will be formulated in solutions or suspensions for topical ophthalmic or intraocular administration, or as tablets, capsules or solutions for systemic administration (e.g., oral or intravenous).

Oral formulations of the JNK inhibitors are preferred due to ease of administration. Oral formulations may be in liquid or solid form. In general, oral formulations will the active JNK inhibitor and inert excipients. In general, solid tablet or capsule dosages will contain various excipients such as bulking agents, binding agents, time release coatings, and so on. Liquid dosages will contain carriers, buffers, tonicity agents, solubilizing agents, and so on.

In general, the doses utilized for the above described purposes will vary, but will be in an effective amount to inhibit or ameliorate retinal neuropathy. As used herein, the term "pharmaceutically effective amount" refers to that amount which inhibits or ameliorates retinal neuropathy. The JNK inhibitors will normally be contained in these formulations in an amount from about 0.01 to about 10.0 weight/percent. Preferable concentrations range from about 0.1 to about 5.0 weight/percent. For topical administration, these formulations are delivered to the disease site one to six times a day, depending on the routine discretion of the skilled clinician. Systemic administration, for example, in the form of tablets or liquid useful for the treatment will contain about 10-1000 mg of a JNK inhibitor, and can be taken 1-4 times per day depending on the discretion of the skilled clinician.

As used herein, the term "pharmaceutically acceptable carrier" refers to any formulation which is safe, and provides the appropriate delivery for the desired route of administration of an effective amount of at least one JNK inhibitor of the present invention.

The following examples are included to demonstrate preferred embodiments of the invention. It should be appreciated by those of skill in the art that the techniques disclosed in the examples which follow represent techniques discovered by the inventor to function well in the practice of the invention, and thus can be considered to constitute preferred modes for its practice. However, those of skill in the art should, in light of the present disclosure, appreciate that many changes can be made in the specific embodiments which are disclosed and still obtain a like or similar result without departing from the spirit and scope of the invention.

### Example 1

The following example demonstrates the protective efficacy of a JNK inhibitor against cytotoxic insults to retinal cells.

### Rat Retinal Ganglion Cell Survival Assay

Adult Sprague-Dawley rats were euthanized by CO₂ asphyxiation. Their eyes were enucleated and placed in Dulbecco's modified Eagle's medium: Nutrient mixture F12 (1:1; DMEM/F12). The retinas were incubated in a papain solution, containing papain (34 units/mL), DL-cysteine (3.3 mM), and bovine serum albumin (0.4 mg/ml) in DMEM/F12, for 25 min at 37°C. Retinal pieces were then triturated until cells were dispersed. Cell suspension (1.5 ml; containing approximately 4.5 x 10⁶ cells) was placed into each of the poly-D-lysine coated glass bottom culture dishes. The cells were cultured in a culture medium previously described by Barres *et al.* (1988) for 3 days in 95% air/5% CO₂ at 37°C.

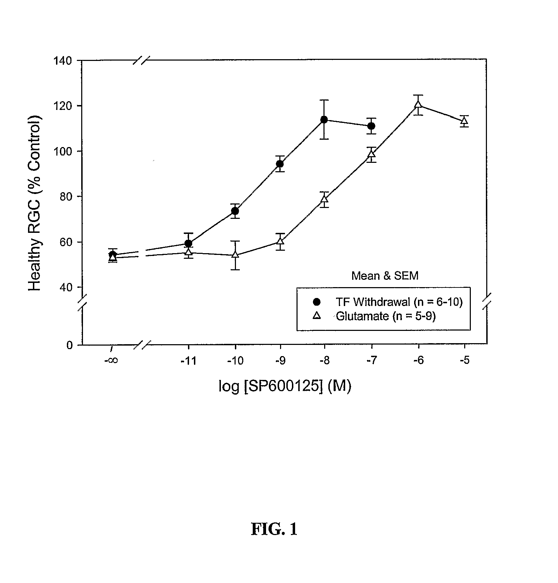

In experiments assessing the toxicity of glutamate on cell survival, the cells were cultured with 100 µM glutamate for 3 days. In experiments assessing the detrimental effect of neurotrophic factor withdrawal on cell survival, basic fibroblast growth factor, brain-derived trophic factor, and ciliary-derived neurotrophic factor were removed from the medium and cells cultured for 3 days. In experiments assessing the potential protective effects of a JNK inhibitor, SP600125, the cells were cultured with the compound in the presence of the glutamate or in the absence of the indicated trophic factors for 3 days. At the end of the 3-day culture period, the cells were immunostained for Thy-1, a cell surface marker for RGC, and observed under a fluorescent microscope. Thy-1-positive cells were counted and averaged. The results are illustrated in FIG. 1.

FIG. 1 illustrates that the survival of RGC depended on the presence of the indicated neurotrophic factors, such that removal of the neurotrophic factors (TF Withdrawal) from the culture medium caused death of RGC to approximately 50% of the control group. Incubation of the cells with SP600125 significantly and completely protected the cells against such insult. FIG. 1 also shows that glutamate was toxic to the RGC, since addition of 100 µM glutamate to the culture medium decreased cell survival by approximately 50%. Again, incubation of the cells with SP600125 also significantly and completely protected the cells against this cytotoxicity.

### Example 2

The following example demonstrates the protective efficacy of a JNK inhibitor against ischemia-induced optic neuropathy in the rat.

### Ischemia/Reperfusion-Induced Optic Neuropathy in the Rat

Adult Wistar rats were anesthetized and the anterior chamber of one eye of each animal was cannulated. The cannula was connected to a raised saline reservoir whose height was adjusted to produce an ocular pressure that was higher than the systolic pressure of the animal, which, by stopping retinal blood flow, produced retinal ischemia. After 60 minutes of ischemia, the intracameral cannula was removed to allow reperfusion of the retina. Two weeks later, the rats were euthanized, their optic nerves isolated, fixed in 2% paraformaldehyde, 2.5% glutaraldehyde in 0.1 M cacodylate buffered solution, sectioned, and stained in 1% *p*-phenylenediamine in isopropanol:methanol (1:1) prepared as described by Hollander and Vaaland (1968). The optic nerve damage in each optic nerve section was ranked by an Optic Nerve Damage Score as previously reported by Pang *et al.* (1999). In this ranking system, a score of 1 represented no damage, and a score of 5 represented total damage.

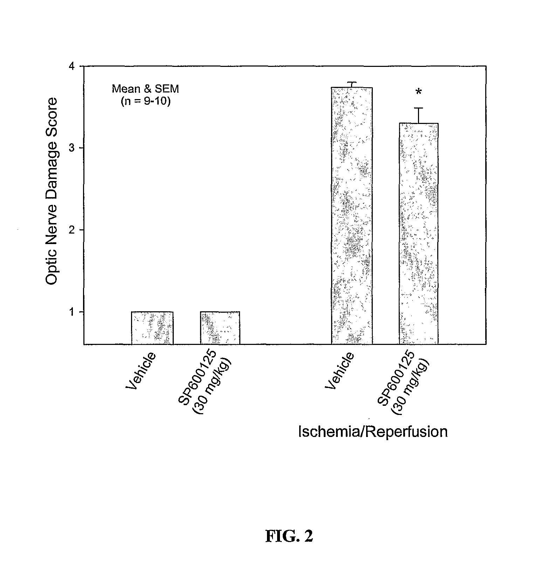

To test the potential protective effect of SP600125, selected animals were treated with a daily intraperitoneal injection of SP600125 (30 mg/kg) for 16 consecutive days starting 2 days before ischemia was induced. The results are illustrated in FIG. 2

FIG. 2 shows that ischemia/reperfusion caused significant damage to the optic nerve as indicated by a dramatic increase in the optic nerve damage score. It also demonstrates that systemic administration of SP600125 could protect against this ischemic insult to the retina as shown by a significant reduction in the optic nerve damage score.

### Example 3

A JNK inhibitor, SP600125, was tested in cultured adult rat retinal ganglion cells (RGC). It was shown to protect against both glutamate-induced and trophic factor withdrawal-induced cytotoxicity.

### METHODS

### A. RGC culture

Adult Sprague-Dawley rats were euthanized by CO₂ asphyxiation. Their eyes were enucleated and the retinas isolated. Retinal cells were treated with of papain solution for 25 min at 37°C, then washed 3 times with 5 mL RGC culture medium (Neurobasal medium with various nutrient supplements + 1% fetal calf serum). Retinal cells were dispersed by trituration. Cell suspension was placed onto poly-D-lysine- and laminin-coated 8-well chambered culture slides. The cells were then cultured in 95% air/5% CO₂ at 37°C.

### B. Cytotoxic Insults

For glutamate-induced toxicity studies, cells were pre-treated with vehicle or the indicated compounds for 30 minutes, followed by 100 µM glutamate for 3 days.

For trophic factor withdrawal studies, three trophic factors, basic fibroblast growth factor, brain-derived neurotrophic factor, and ciliary neurotrophic factor, were removed from the culture medium. Cells were cultured in this medium with the indicated compounds for 3 days.

### C. Quantification of cell survival

At the end of the incubation period, the cells were fixed and labeled for Thy-1, a RGC marker, by immunocytochemistry. Cell survival was quantified by manually counting Thy-1-positive healthy cells in each well.

### RESULTS

### A. Effect of SP600125 on glutamate-induced toxicity in rat RGC

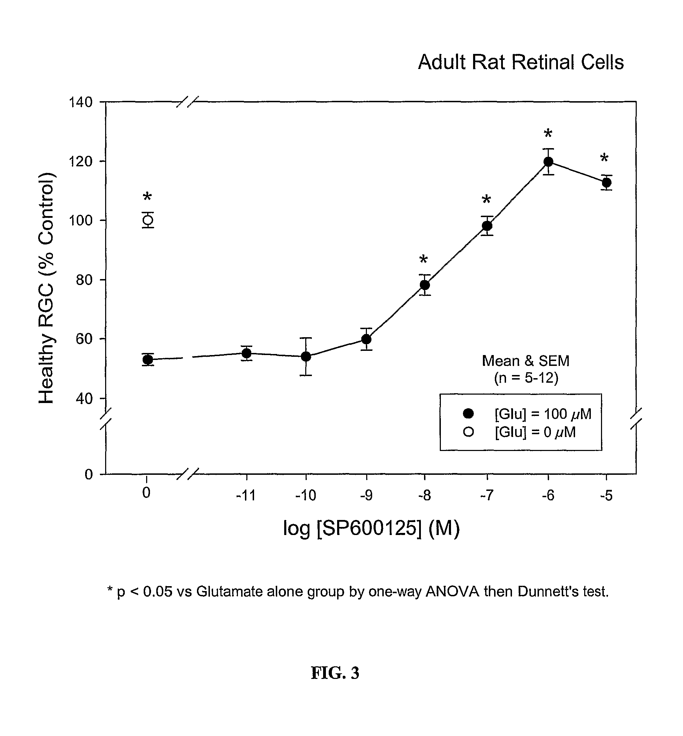

It has been previously shown that glutamate was toxic to rat RGC; only 50-70% of cells survived after a 3-day treatment of 100 µM glutamate. The glutamate-induced toxicity in these cells can be prevented by pretreatment with MK801. SP600125 was protective against this insult in a dose-dependent manner (FIG. 3).

### B. Effect of SP600125 on trophic factor withdrawal-induced toxicity in RGC

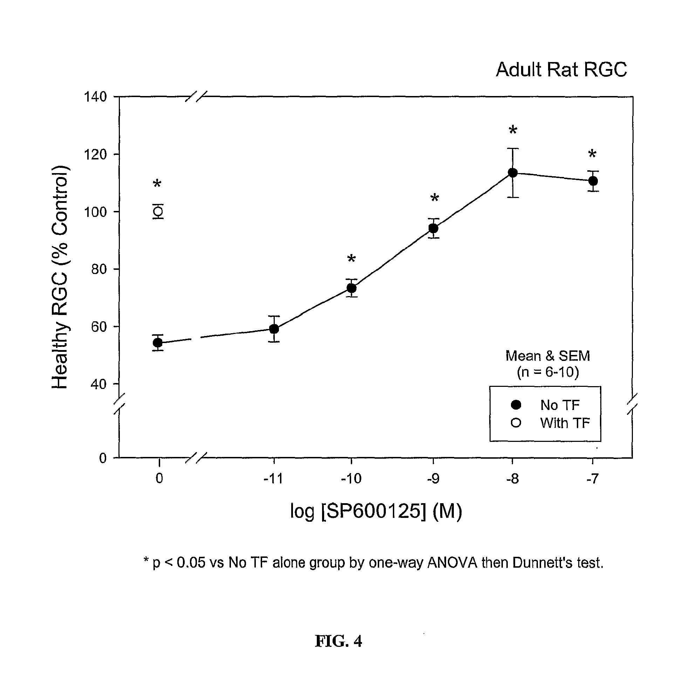

Previously, it was shown that removal of the three trophic factors for 3 days caused death of approximately 40-50% of the cells. SP600125 was protective against this insult in a dose-dependent manner (FIG. 4).

### Example 4

Topical compositions useful for treating glaucoma and other ocular diseases:

| Component | Wt.% |
|---|---|
| JNK inhibitor | 0.1-5 |
| HPMC | 0.01-10 |
| Benzalkonium Chloride | 0.005-0.5 |
| Sodium Chloride | 0.5-2.0 |
| Edetate Disodium | 0.005-0.5 |
| NaOH/HCl | q.s. pH 7.4 |
| Purified Water | q.s. 100 mL |

The above formulation is prepared by first placing a portion of the purified water into a beaker and heating to 90°C. The hydroxypropylmethylcellulose (HPMC) is then added to the heated water and mixed by means of vigorous vortex stirring until all of the HPMC is dispersed. The resulting mixture is then allowed to cool while undergoing mixing in order to hydrate the HPMC. The resulting solution is then sterilized by means of autoclaving in a vessel having a liquid inlet and a hydrophobic, sterile air vent filter.

The sodium chloride and the edetate disodium are then added to a second portion of the purified water and dissolved. The benzalkonium chloride is then added to the solution, and the pH of the solution is adjusted to 7.4 with 0.1M NaOH/HCl. The solution is then sterilized by means of filtration.

SP600125 is sterilized by either dry heat or ethylene oxide. If ethylene oxide sterilization is selected, aeration for at least 72 hours at 50°C is necessary. The sterilized compound is weighed aseptically and placed into a pressurized ballmill container. Sterilized glass balls are then added to the container and the contents of the container are milled aseptically at 225 rpm for 16 hours, or until all particles are in the range of approximately 5 microns.

Under aseptic conditions, the micronized drug suspension or solution formed by means of the preceding step is then poured into the HPMC solution with mixing. The ballmill container and balls contained therein are then rinsed with a portion of the solution containing the sodium chloride, the edetate disodium and benzalkonium chloride. The rinse is then added aseptically to the HPMC solution. The final volume of the solution is then adjusted with purified water and, if necessary, the pH of the solution is adjusted to pH 7.4 with NaOH/HCl.

### Example 5

### Preferred formulation for topical administration:

| Component | Wt.% |
|---|---|
| SP600125 | 0.1-5 |
| HPMC | 0.5 |
| Benzalkonium Chloride | 0.01 |
| Sodium Chloride | 0.8 |
| Edetate Disodium | 0.01 |
| NaOH/HCl | q.s. pH 7.4 |
| Purified Water | q.s. 100 mL |

### Example 6

### Formulation for oral administration:

### Tablet:

1-1000 mg of a JNK inhibitor with inactive ingredients such as starch, lactose and magnesium stearate can be formulated according to procedures known to those skilled in the art of tablet formulation.

All of the compositions and/or methods disclosed and claimed herein can be made and executed without undue experimentation in light of the present disclosure. While the compositions and methods of this invention have been described in terms of preferred embodiments, it will be apparent to those of skill in the art that variations may be applied to the compositions and/or methods and in the steps or in the sequence of steps of the method described herein without departing from the concept, spirit and scope of the invention. More specifically, it will be apparent that certain agents which are both chemically and structurally related may be substituted for the agents described herein to achieve similar results. All such substitutions and modifications apparent to those skilled in the art are deemed to be within the spirit, scope and concept of the invention as defined by the appended claims.

### References

The following references, to the extent that they provide exemplary procedural or other details supplementary to those set forth herein, are specifically incorporated herein by reference.

### Patents and Published Patent Applications

WO200035906
WO200035909
WO200035921
WO200064872
W0200112609
WO20011262
WO200123378
W0200123379
W0200123382
W0200147920
W0200191749
W02002046170
WO2002062792
W02002081475
WO2002083648
WO2003024967

### Books

### Other Publications

Adjei, "Blocking oncogenic Ras signaling for cancer therapy," J NATL CANCER INST 93:1062-1074 (2001).
Amendola et al., "Postnatal changes in nerve growth factor and brain derived neurotrophic factor levels in the retina, visual cortex, and geniculate nucleus in rats with retinitis pigmentosa," NEUROSCI LETT 345:37-40 (2003).
Barres et al., "Immunological, morphological, and electrophysiological variation among retinal ganglion cells purified by panning," NEURON 1:791-803 (1988).
Bruckner et al., "JNK3 contributes to c-Jun activation and apoptosis but not oxidative stress in nerve growth factor-deprived sympathetic neurons," J NEUROCHEM 78:298-303 (2001).
Clark & Pang, "Advances in Glaucoma Therapeutics," EXPERT OPIN EMERGING DRUGS 7:141-164 (2002).
Gupta et al., "Selective interaction of JNK protein kinase isoforms with transcription factors," EMBO J 15:2760-2770 (1996).
Harris et al., "Progress in measurement of ocular blood flow and relevance to our understanding of glaucoma and age-related macular degeneration," PROG RETINA EYE RES 18:669-687 (1999).
Hirosumi et al, "A central role for JNK in obesity and insulin resistance," NATURE 420:333-337 (2002).
Hisatomi et al., "Critical role of photoreceptor apoptosis in functional damage after retinal detachment," CURR EYE RES 24:161-172 (2002).
Hollander and Vaaland, "A reliable staining method for semi-thin sections in experimental neuroanatomy," BRAIN RES 10:120-126 (1968).
Le-Niculescu et al., "Withdrawal of survival factors results in activation of the JNK pathway in neuronal cells leading to Fas ligand induction and cell death," MOL CELL BIOL 19:751-763 (1999).
Lewis et al., "Effects of neurotrophin brain-derived neurotrophic factor in an experimental model of retinal detachment," INVEST OPHTHALMOL VIS SCI 40:1530-1544 (1999).
Manning & Mercurio, "Transcription inhibitors in inflammation," EXP OPIN INVEST DRUGS 6:555-567 (1997).
Pang et al., "Age-dependent changes in ocular morphology of a spontaneous ocular hypertetzsive mouse strain (DBA/2J)," INVEST OPHTHALMOL VIS RES 40:S671 (1999).
Schumer et al., "The nerve of glaucoma!," ARCH OPHTHALMOL 112:37-44 (1994).
Sherry & Townes-Anderson, "Rapid glutamtergic alterations in the neural retinal induced by retinal detachment," INVEST OPHTHALMOL VIS SCI 41:2779-2790 (2000).
Tao et al., "Encapsulated cell-based delivery of CNTF reduces photoreceptor degeneration in anima models of retinitis pigmentosa," INVEST OPHTHALMOL VIS SCI 43:3292-3298 (2002).
Tournier et al., "Requirement of JNK for stress-induced activation of the cytochrome c-mediated death pathway," SCIENCE 288:870-874 (2000).
Van Buskirk et al., "Predicted outcome from hypotensive therapy for glaucomatous optic neuropathy," AM J OPHTHALMOL 25:636-640 (1993).
Xia et al., "Opposing effects of ERK and JNK-p38 MAP kinases on apoptosis," SCIENCE 270:1326-1331 (1995).
Yamada et al., "Fibroblast growth factor-2 decreases hyperoxia-induced photoreceptor cell death in mice," AM J PATHOL 159:1113-1120 (2001).
Yang et al., "Absence of excitotoxicity-induced apoptosis in the hippocampus of mice lacking the JNK3 gene," NATURE 389:865-870 (1997).

The invention further provides the following:

A composition for the treatment of glaucoma and other ocular diseases, such as acute ischemic optic neuropathy, macular degeneration, retinitis pigmentosa, retinal detachment, retinal tears or holes, and other ischemic retinopathies or optic neuropathies, comprising an effective amount of one or more inhibitors of Jun N-terminal kinases (JNK) and a pharmaceutically acceptable vehicle.

A composition as above, wherein the JNK inhibitor is SP600125.

A composition as above, wherein the composition is an oral formulation.

A composition as above, wherein the composition is a topical ophthalmic, surgical irrigating solution, or an intraocular formulation.

A composition as above, wherein the composition is an oral formulation.

A composition as above, wherein the composition is a topical ophthalmic, surgical irrigating solution or an intraocular formulation.

A method for the treatment of glaucoma which comprises administering to a human patient a composition comprising an effective amount of one of more JNK inhibitor(s) and a pharmaceutically acceptable vehicle.

A method as above, wherein the JNK inhibitor is SP600125.

A method as above, wherein the composition is an oral formulation.

A method as above, wherein the composition is a topical ophthalmic, surgical irrigating solution or an intraocular formulation.

A method as above wherein the composition is an oral formulation.

A method as above, wherein the composition is a topical ophthalmic, surgical irrigating solution or an intraocular formulation.

A method for treating ocular diseases selected from the group consisting of acute ischemic optic neuropathy, macular degeneration, retinitis pigmentosa, retinal detachment, retinal tears or holes, and other ischemic retinopathies or optic neuropathies, said method comprising administering to a human patient a composition comprising an effective amount of one or more JNK inhibitor(s) and a pharmaceutically acceptable vehicle.

A method as above, wherein the JNK inhibitor is SP600125.

A method as above, wherein the composition is an oral formulation.

A method as above, wherein the composition is a topical ophthalmic, surgical irrigating solution or an intraocular formulation.

A method as above, wherein the composition is an oral formulation.

A method as above, wherein the composition is a topical ophthalmic, surgical irrigating solution or an intraocular formulation.

## Claims

1. A composition for the treatment of glaucoma and other ocular diseases, such as acute ischemic optic neuropathy, macular degeneration, retinitis pigmentosa, retinal detachment, retinal tears or holes, and other ischemic retinopathies or optic neuropathies, comprising an effective amount of one or more inhibitors of Jun-N-terminal kinases (JNK) and a pharmaceutically acceptable vehicle.

2. A composition according to Claim 1, wherein the JNK inhibitor is SP600125.

3. A composition according to Claim 1, wherein the composition is an oral formulation.

4. A composition according to claim 1, wherein the composition is a topical ophthalmic, surgical irrigating solution, or an intraocular formulation.

5. Use of one or more JNK inhibitor(s) for treating ocular diseases selected from the group consisting of acute ischemic optic neuropathy, macular degeneration, retinitis pigmentosa, retinal detachment, retinal tears or holes, and other ischemic retinopathies or optic neuropathies, in the preparation of a medicament.
